(19) Europäisches Patentamt
European Patent Office
Office européen des brevets

(11) Veröffentlichungsnummer: **0 261 604 B1**

(12) **EUROPÄISCHE PATENTSCHRIFT**

(45) Veröffentlichungstag der Patentschrift: **22.01.92**

(21) Anmeldenummer: **87113688.3**

(22) Anmeldetag: **18.09.87**

(51) Int. Cl.⁵: **C07C 265/04**, C07C 263/06, C07C 271/04, C07C 271/06, C07C 269/06, C08G 18/77

# (54) Neue 2-(Alkoximethyl)-pentan-1,5-diisocyanate, - diurethane und dicarbamidsäurechloride, Verfahren zu ihrer Herstellung und deren Verwendung.

(30) Priorität: **20.09.86 DE 3632010**

(43) Veröffentlichungstag der Anmeldung: **30.03.88 Patentblatt 88/13**

(45) Bekanntmachung des Hinweises auf die Patenterteilung: **22.01.92 Patentblatt 92/04**

(84) Benannte Vertragsstaaten: **BE CH DE ES FR GB IT LI NL**

(56) Entgegenhaltungen:
**EP-A- 0 018 586**
**EP-A- 0 077 105**
**DE-A- 1 908 569**
**DE-A- 3 517 110**
**US-A- 3 631 198**

(73) Patentinhaber: **BASF Aktiengesellschaft**
**Carl-Bosch-Strasse 38**
**W-6700 Ludwigshafen(DE)**

(72) Erfinder: **Merger, Franz, Dr.**
**Max-Slevogt-Strasse 25**
**W-6710 Frankenthal(DE)**
Erfinder: **Schwarz, Wolfgang, Dr.**
**Wesostrasse 132**
**W-7507 Pfinztal(DE)**

**EP 0 261 604 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue 2-(Alkoximethyl)-pentan-1,5-diisocyanate, Verfahren zu ihrer Herstellung und deren Verwendung, vorzugsweise zur Herstellung von Kunststoffen nach dem Polyisocyanat-polyadditionsverfahren, sowie 2-(Alkoximethyl)-pentan-1,5-diurethane und -dicarbamidsäurechloride, die sich insbesondere als Ausgangsstoffe zur Herstellung der erfindungsgemäßen 2-(Alkoximethyl)-pentan-1,5-diisocyanate eignen.

Der Erfindung liegt die Aufgabe zugrunde, neue wirtschaftlich herstellbare und technisch vorteilhaft verarbeitbare aliphatische Diisocyanate, die als Zwischenprodukte insbesondere zur Herstellung von Kunststoffen nach dem Polyisocyanat-polyadditionsverfahren wertvoll sind, sowie ein Verfahren zu ihrer Herstellung zur Verfügung zu stellen.

Diese Aufgabe wird gelöst mit 2-(Alkoximethyl)-pentan-1,5-diisocyanaten der Formel (I)

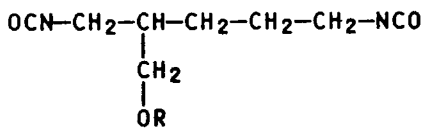

(I),

in der R bedeutet:
einen geradkettigen oder verzweigten $C_1$-$C_{20}$-Alkylrest, vorzugsweise $C_1$-$C_{12}$-Alkylrest,
einen geradkettigen oder verzweigten $C_2$-$C_{20}$-Alkenylrest, vorzugsweise $C_2$-$C_{12}$-Alkenylrest,
einen geradkettigen oder verzweigten $C_3$-$C_{20}$-Oxaalkylrest, vorzugsweise $C_3$-$C_{12}$-Oxaalkylrest,
einen $C_5$-$C_{12}$-Cycloalkylrest, vorzugsweise unsubstituierten $C_6$-$C_{12}$-Cycloalkylrest oder
einen $C_7$-$C_{20}$-Aralkylrest, vorzugsweise unsubstituierten $C_7$-$C_{12}$-Aralkylrest.

Besonders bewährt haben sich und daher insbesondere Anwendung finden Diisocyanate der Formel (I), in der R ausgewählt ist unter Methyl, Ethyl, n-Propyl, i-Propyl, n-Butyl, i-Butyl, sec.-Butyl, 2-Methylbutyl, n-Pentyl, Neopentyl, 2-Methylpentyl, n-Hexyl, 2-Ethylhexyl, n-Octyl, n-Decyl, n-Dodecyl, Cyclohexyl, Benzyl, Phenylethyl, Methoxiethyl, Ethoxiethyl, Butoxiethyl und Isobutoxiethyl.

Besonders bevorzugte erfindungsgemäße Verbindungen der allgemeinen Formel I sind die folgenden:
2-(Methoximethyl)-pentan-1,5-diisocyanat,
2-(Ethoxim thyl)-pentan-1,5-diisocyanat,
2-(n-Propoximethyl)-pentan-1,5-diisocyanat,
2-(Isopropoximethyl)-pentan-1,5-diisocyanat,
2-(n-Butoximethyl)-pentan-1,5-diisocyanat,
2-(2-Methylbutoximethyl)-pentan-1,5-diisocyanat,
2-(Neopentoximethyl)-pentan-1,5-diisocyanat,
2-(2-Methylpentoximethyl)-pentan-1,5-diisocyanat,
2-(n-Hexoximethyl)-pentan-1,5-diisocyanat,
2-( n-Dodecoximethyl)-pentan-1,5-diisocyanat,
2-(n-Cyclohexoximethyl)-pentan-1,5-diisocyanat,
2-(2-Methoxiethoximethyl)-pentan-1,5-diisocyanat,
2-(2-Ethoxiethoximethyl)-pentan-1,5-diisocyanat,
2-(2-Butoxiethoximethyl)-pentan-1,5-diisocyanat, 2-(2-Isobutoxiethoximethyl)-pentan-1,5-diisocyanat.

Die Verbindungen der Formel (I) sind erhältlich durch thermische Spaltung von 2-(Alkoximethyl)-pentan-1,5-dicarbamidsäurechloriden der Formel III

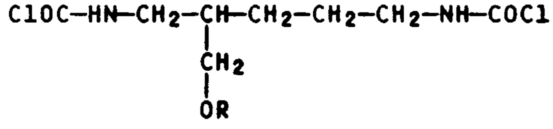

(III)

in der R die vorgenannte Bedeutung besitzt, in Gegenwart von unter den Reaktionsbedingungen gegenüber NCO-Gruppen inerten organischen Lösungsmitteln bei Temperaturen von 80 bis 200 °C, vorzugsweise 120 bis 200 °C in die erfindungsgemäßen 2-(Alkoximethyl)-pentan-1,5-diisocyanate und Chlorwasserstoff.

Vorzugsweise werden die 2-(Alkoxyimethyl)-pentan-1,5-diisocyanate der Formel (I) jedoch hergestellt durch thermische Spaltung von 2-(Alkoximethyl)-pentan-1,5-diurethanen der Formel (II)

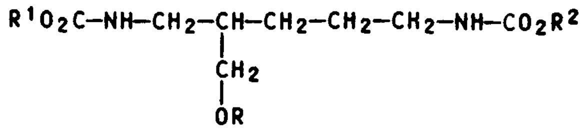 (II) ,

in der R die obengenannte Bedeutung besitzt und $R^1$ und $R^2$ gleich oder verschieden sind und bedeuten einen geradkettigen oder verzweigten $C_1$-$C_{20}$-Alkylrest, vorzugsweise $C_1$-$C_{10}$-Alkylrest und insb esondere $C_3$-$C_6$-Alkylrest oder einen $C_3$-$C_{15}$-Cycloalkylrest, vorzugsweise $C_5$-$C_{10}$-Cycloalkylrest und insbesondere $C_5$-$C_8$-Cycloalkylrest, in Gegenwart oder Abwesenheit von Katalysatoren

a) in der Gasphase bei Temperaturen von über 300 ° C unter vermindertem Druck oder

b) in flüssiger Phase bei Temperaturen von 175 bis 350 ° C

in die erfindungsgemäßen 2-(Alkoximethyl)-pentan-1,5-diisocyanate und Alkohol.

Die 2-(Alkoximethyl)-pentan-1,5-diurethane der Formel (II) und -dicarbamidchloride der Formel (III) sind erhältlich durch Umsetzung von 2-(Alkoximethyl)-pentan-1,5-diaminen der Formel (IV)

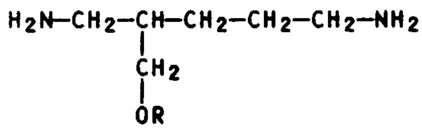 (IV),

in der R die vorgenannte Bedeutung besitzt, in Gegenwart oder Abwesenheit von Katalysatoren mit

a) Harnstoff und einem primären und/oder sekundären Alkohol $R^1OH$ bzw. $R^2OH$ oder

b) Harnstoff und einem primären und/oder sekundären Alkohol $R^1OH$ bzw. $R^2OH$, wobei die Reste $R^1$ und $R^2$ die obengenannte Bedeutung haben, in Gegenwart von Carbamidsäurealkylestern und/oder Dialkylcarbonaten

und gegebenenfalls Abtrennung des gebildeten Ammoniaks bzw. durch Phosgenierung der 2-(Alkoximethyl)-pentan-1,5-diamine der Formel (IV) oder deren Salze, vorzugsweise der 2-(Alkoximethyl)-pentan-1,5-diaminhydrochloride, in Lösungs- oder Verdünnungsmitteln.

Die neuen erfindungsgemäßen 2-(Alkoximethyl)-pentan-1,5-diisocyanate sowie die Ausgangsstoffe 2-(Alkoximethyl)-pentan-1,5-diurethane und -dicarbamidsäurechloride zu deren Herstellung und die Vorprodukte hierfür können beispielsweise nach folgenden Verfahren hergestellt werden:

2-(Alkoximethyl)-pentan-1,5-diamine der Formel (IV) sind z.B. dadurch erhältlich, daß man 2-(Alkoximethyl)-glutarsäuredinitrile der Formel (V)

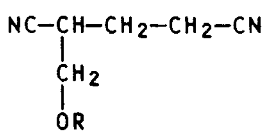 (V)

in der R die obengenannte Bedeutung besitzt, und die nach DE-A-15 93 176 (GB-A-1 097 360) in guten Ausbeuten aus 2-Methylenglutarsäuredinitril und Alkohol zugänglich sind, gegebenenfalls in Gegenwart eines Katalysators bei erhöhter Temperatur unter Druck hydriert.

Nach einer bevorzugten Ausführungsform hydriert man die 2-(Alkoximethyl)-glutarsäuredinitrile der Formel (V) gegebenenfalls in einem inerten Lösungsmittel, in Gegenwart von Wasserstoff und Ammoniak bei einer Temperatur zwischen 50 und 200 ° C in Gegenwart eines Hydrierkatalysators, insbesondere mit einem Metall der 8. Nebengruppe des Periodensystems der Elemente unter einem Druck von 1 bis 300 bar.

Die Hydrierung der 2-(Alkoximethyl)-glutarsäuredinitrile der Formel (V) zu 2-(Alkoxiethyl)-pentan-1,5-diaminen der Formel (V) kann prinzipiell in an sich bekannter Weise erfolgen z.B. durch katalytische Hydrierung, durch Umsetzen mit Lithiumaluminiumhydrid im inerten, trockenen Lösungsmittel oder mit Natrium in Gegenwart von Alkoholen; bevorzugt ist die katalytische Hydrierung an Katalysatoren aus der 8. Nebengruppe des Periodensystems.

Die katalytische Hydrierung erfolgt vorzugsweise - wie bereits dargelegt wurde - durch Erhitzen der Verbindung der allgemeinen Formel V in einem bei der Reaktion inerten Lösungsmittel auf eine Temperatur von 50 bis 200 ° C, bevorzugt 100 bis 180 ° C, in Gegenwart von Wasserstoff, Ammoniak und üblichen Hydrierkatalysatoren, bevorzugt aus der 8. Nebengruppe des Periodensystems. Ammoniak sollte zweckmäßig stöchiometrisch oder bevorzugt im Überschuß vorliegen, z.B. im Molverhältnis 2:1 bis 40:1, bezogen auf eingesetztes Dinitril, bevorzugt im Molverhältnis 5:1 bis 25:1.

Die Reaktion wird zweckmäßig unter einem Druck von 1 bis 300 bar, bevorzugt 50 bis 180 bar Wasserstoff durchgeführt.

Die Aufarbeitung erfolgt in üblicher Weise, z.B. durch Abfiltrieren des Katalysators, Destillation und/oder Kristallisation des Rückstandes.

Die Reaktion kann diskontinuierlich, aber auch kontinuierlich durchgeführt werden. Als Reaktoren sind z.B. einfache Stahlautoklaven geeignet, für kontinuierliche Hydrierungen druckfeste, mit Festbettkatalysatoren gefüllte Stahlrohre.

Als Hydrierkatalysatoren eignen sich alle für Nitrilhydrierungen üblichen Katalysatoren, bevorzugt aus der 8. Nebengruppe des Periodensystems, wie z.B. Nickel-, Kobalt- oder Eisen-Katalysatoren, aber auch Edelmetallkatalysatoren wie Palladium, Platin, Ruthenium oder Rhodium. Die Katalysatormetalle können als Vollkatalysatoren, z.B. in feiner Verteilung wie Raney-Nickel oder Raney-Kobalt in Suspensionsfahrweise, als geformte metallische Eisenpigmentkatalysatoren, als Mischkatalysatoren oder auf Trägern niedergeschlagen, angewandt werden. Als Träger eignen sich beispielsweise Aluminiumoxid, Kieselgel oder Magnesiumsilikate. Besonders bevorzugt sind Raney-Kobalt und Eisenpigment-Katalysatoren.

Der Katalysator wird bei diskontinuierlicher Fahrweise in Mengen von 1 bis 100 Gew.%, bevorzugt 10 bis 50 Gew.%, bezogen auf die verwendete Menge an Verbindung der allgemeinen Formel V, eingesetzt.

Als Lösungsmittel für die katalytische Hydrierung können alle unter den Reaktionsbedingungen inerten Lösungsmittel verwendet werden, bevorzugt sind Tetrahydrofuran, Dioxan und Alkohole wie Methanol, Ethanol, Propanol oder Butanol oder Gemische daraus. Auch flüssiges Ammoniak eignet sich als Lösungsmittel. Das Lösungsmittel wird in einer Menge von 100 bis 10.000 Gew.%, bevorzugt 200 bis 2.000 Gew.%, bezogen auf den Ausgangsstoff der allgemeinen Formel V, eingesetzt.

Andere Methoden zur Nitrilhydrierung sind ebenso geeignet, z.B. die Reduktion mit komplexen Aluminiumhydriden (Lithiumaluminiumhydrid) in wasserfreien inerten Lösungsmitteln wie Ether oder Tetrahydrofuran, oder die Reduktion mit Natrium in Alkoholen wie z.B. in Ethanol, Propanol oder n-Butanol. Die Wahl der Reduktionsmethode ist von der Bedeutung des Restes R in den Verbindungen der allgemeinen Formel II abhängig; für R = Alkenyl sind katalytische Hydrierungen beispielsweise weniger geeignet.

Nach dem beschriebenen Verfahren, das Gegenstand der DE-A-36 32 007 ist, werden z.B. vorzugsweise folgende 2-(Alkoximethyl)-pentan-1,5-diamine der Formel (IV) hergestellt:

2-(Methoximethyl)-pentan-1,5-diamin
2-(Ethoximethy )-pentan-1,5-diamin
2-(n-Propoximethyl)-pentan-1,5-diamin
2-(Isopropoximethyl)-pentan-1,5-diamin
2-(n-Butoximethyl -pentan-1,5-diamin
2-(2-Methylbutoximethyl)-pentan-1,5-diamin
2-(Neopentoximethyl)-pentan-1,5-diamin
2-(2-Methylpentoximethyl)-pentan-1,5-diamin
2-(n-Hexoximethyl)-pentan-1,5-diamin
2-(2-Ethylhexoximethyl)-pentan-1,5-diamin
2-(n-odecoximethyl)-pentan-1,5-diamin
2-(n-Cyclohexoximethyl)-pentan-1,5-diamin
2-(2-Methoxiethoximethyl)-pentan-1,5-diamin 2-(2-Ethoxiethoximethyl)-pentan-1,5-diamin
2-(2 -Butoxiethoximethyl)-pentan-1,5-diamin und
2-(2-Isobutoxiethoximethyl)-pentan-1,5-diamin

Zur Herstellung der 2-(Alkoximethyl)-pentan-1,5-dicarbamidsäurechloride der Formel (III) können die 2-(Alkoximethyl)-pentan-1,5-diamine direkt oder als Salze, vorzugsweise als Hydrochloride in Lösungs- oder Verdünnungsmitteln nach bekannten Methoden phosgeniert werden. Geeignete Lösungsmittel sind beispielsweise Toluol, Xylol, Chlorbenzol, Dichlorbenzol oder Mono- und/oder Dicarbonsäureester mit Siedepunkten von 165 bis 250 °C, wie z.B. Benzoesäuremethylester, Oxalsäure- und/oder Adipinsäuredimethylester. Eine Lösung der 2-(Alkoximethyl)-pentan-1,5-diamine oder eine Suspension der entsprechenden Salze wird danach bei Temperaturen von ungefähr 0 bis 80 °C, vorzugsweise 10 bis 50 °C mit 1 bis 6 Mol, vorzugsweise 1 bis 2,5 Mol, und insbesondere 1 bis 1,5 Mol Phosgen pro $-NH_2$- oder $-NH_2•HCl$-Gruppe zur Reaktion gebracht. Das gasformige oder flüssige Phosgen wird hierbei der Reaktionsmischung mit einer solchen Geschwindigkeit zugeführt, daß die austretenden Gase überwiegend aus Chlorwasserstoff bestehen. Durch Abtrennung des Lösungsmittels, beispielsweise durch Destillation unter Normal- oder vermindertem Druck können die 2-(Alkoximethyl )-pentan-1,5-dicarbamidsäurechloride isoliert und nach bekannten Methoden gereinigt werden.

Vorzugsweise werden jedoch die gebildeten 2-(Alkoximethyl)-pentan-1,5-dicarbamidsäurechloride ohne intermediäre Isolierung in den beispielhaft genannten Lösungsmitteln bei Temperaturen von 80 bis 200 °C, vorzugsweise von 120 bis 180 °C in die erfindungsgemäßen 2-(Alkoximethyl)-pentan-1,5-diisocyanate der Formel (I) und Chlorwasserstoff gespalten.

Nach beendeter Phosgenierung und Spaltung wird das Lösungsmittel, vorzugsweise unter vermindertem Druck, beispielsweise von 100 bis 5 mbar abdestilliert. Gegebenenfalls kann es auch vorteilhaft sein, den Chlorwasserstoff und evtl. vorliegendes überschüssiges Phosgen mit Hilfe von Stickstoff oder einem anderen Inertgas aus der Diisocyanatlösung auszutreiben, bevor das Lösungsmittel abdestilliert wird.

Die erhaltenen rohen 2-(Alkoximethyl)-pentan-1,5-diisocyanate können nach bekannten Methoden, beispielsweise durch Destillation unter vermindertem Druck, gereinigt werden.

Nach bevorzugten Ausführungsformen werden die erfindungsgemäßen 2-(Alkoximethyl)-pentan-1,5-diurethane der Formel (II) hergestellt durch Umsetzung von 2-(Alkoximethyl)-pentan-1,5-diaminen der Formel (IV) mit

a) Harnstoff und einem primären und/oder sekundären Alkohol $R^1OH$ bzw. $R^2OH$ oder vorzugsweise

b) Harnstoff und einem primären und/oder sekundären Alkohol $R^1OH$ bzw. $R^2OH$ in Gegenwart von Carbamidsäurealkylestern und/oder Dialkylcarbonaten und

gegebenenfalls Abtrennung des gebildeten Ammoniaks. Die Reaktionen können in Gegenwart oder Abwesenheit von Katalysatoren durchgeführt werden.

Die Diurethane nach Formel II können jedoch auch nach anderen Methoden erhalten werden, wie z.B. durch Umsetzung der Diamine mit Carbamidsäurealkylester nach Angaben der EP-A-18 588 oder mit Dialkylcarbonaten, zweckmäßigerweise in Gegenwart von Alkoholen oder durch Umsetzung der Diamine mit Chlorkohlensäurealkylestern.

Nach den bevorzugt durchgeführten Verfahren zur Herstellung der 2-(Alkoximethyl)-pentan-1,5-diurethane der Formel II werden die 2-(Alkoximethyl)-pentan-1,5-diamine der Formel IV mit Harstoff und Alkohol im Molverhältnis 1:1,5 bis 10:2 bis 50, vorzugsweise 1:2,0 bis 2,5:4 bis 20 und insbesondere 1:2,0 bis 2,3:4 bis 10 in Abwesenheit oder in Gegenwart von Katalysatoren bei Reaktionstemperaturen von 175 bis 250 °C, vorzugsweise 180 bis 230 °C zur Reaktion gebracht. Der im Laufe der Reaktion gebildete Ammoniak wird zweckmäßigerweise sofort abgetrennt. Die Reaktion wird vor allem bei Verwendung niedrig siedender Alkohole unter Druck durchgeführt, wobei der Druck so eingestellt wird, daß das Reaktionsgemisch bei der angewandten Reaktionstemperatur siedet. In Abhängigkeit von dem verwendeten Alkohol beträgt der Druck üblicherweise 0,1 bis 60 bar, vorzugsweise 1 bis 40 bar. Für diese Reaktionsbedingungen ergeben sich Reaktionszeiten von 0,5 bis 50, vorzugsweise 3 bis 15 Stunden.

Als Alkohole $R^1OH$ und $R^2OH$, die gleich oder verschieden sein können, eignen sich prinzipiell beliebige, gegebenenfalls substituierte primäre und/oder sekundäre aliphatische und/oder cycloaliphatische Alkohole. Vorzugsweise wird man jedoch solche auswählen, deren Siedepunkte genügend weit vom Siedepunkt des durch nachfolgende chemische Spaltung erhaltenen 2-(Alkoximethyl)-pentan-1,5-diisocyanats entfernt liegen, so daß einerseits eine möglichst quantitative Trennung der Spaltprodukte Diisocyanat und Alkohol möglich ist und andererseits die gebildeten 2-(Alkoximethyl)-pentan-1,5-diurethane, möglichst unzersetzt verdampft werden können.

Als Alkohole $R^1$ bzw. $R^2OH$ in Betracht kommen beispielsweise aliphatische, gegebenenfalls substituierte primäre oder sekundäre Alkohole mit 1 bis 20 Kohlenstoffatomen, vorzugsweise 1 bis 10 Kohlenstoffatomen oder insbesondere 3 bis 6 Kohlenstoffatomen in einem geradkettigen oder verzweigten Alkylrest und/oder cycloaliphatische, gegebenenfalls substituierte Alk ohole mit 3 bis 15 Kohlenstoffatomen und insbesondere 5 bis 8 Kohlenstoffatomen im gegebenenfalls substituierten Cycloakylrest. Beispielhaft genannt seien Alkohole wie Methanol, Ethanol, Propanol, 2-Phenyl-propanol, n-Butanol, Isobutanol, 2- und 3-Methylbutanol, Neopentylalkohol, Pentanol, 2-Methyl-pentanol, n-Hexanol, 2-Ethylhexanol, n-Heptanol, n-Octanol, n-Nonanol, n-Decanol, n-Dodecanol, Benzylalkohol, Isopropanol, sec.Butanol, sec.-Isoamylalkohol, Cyclopentanol, Cyclohexanol, 2-, 3- oder 4-Methyl-cyclohexanol und tert.-Butylcyclohexanol. Vorzugsweise verwendet werden Methanol, Ethanol, n-Propanol, n-Butanol, iso-Butanol, n-Pentanol, iso-Pentanol, n-Hexanol oder Gemische der aliphatischen und/oder cycloaliphatischen Alkohole sowie insbesondere n-Propanol, n- und/oder iso-Butanol.

Wie bereits dargelegt wurde, kann die Umsetzung der 2-(Alkoxyimethyl)-pentan-1,5-diamine der Formel IV mit Harnstoff und Alkohol auch in Gegenwart von Carbamidsäurealkylester und/oder Dialkylcarbonaten durchgeführt werden. Bei diesen Verfahrensvarianten werden das Dialkylcarbonat zweck mäßigerweise in einer Menge von 1 bis 30 Mol.%, vorzugsweise 5 bis 25 Mol.% oder die Carbamidsäurealkylester in einer Menge von 1 bis 20 Mol.%, vorzugsweise von 5 bis 18 Mol.%, bezogen auf die 2-(Alkoximethyl)-pentan-1,5-diamine eingesetzt. Vorzugsweise verwendet werden jedoch Mischungen aus Dialkylcarbonaten und Carbamidsäurealkylestern in den genannten Mengenverhältnissen. Als Dialkylcarbonate und/oder Carbamidsäurealkylester setzt man bevorzugt solche ein, deren Alkylreste dem Alkylrest des verwendeten Alkohols entsprechen.

Zur Erhöhung der Reaktionsgeschwindigkeit können die 2-(Alkoximethyl)-pentan-1,5-diurethane der Formel (II) in Gegenwart von Katalysatoren hergestellt werden. Diese werden zweckmäßigerweise in Mengen von 0,1 bis 20 Gew.%, vorzugsweise 0,5 bis 10 Gew.%, und insbesondere 1 bis 5 Gew.%, bezogen auf das Gewicht der 2-(Alkoximethyl)-pentan-1,5-diamine verwendet. Als Katalysatoren eignen sich anorganische oder organische Verbindungen, die ein oder mehrere Kationen, vorzugsweise ein Kation von Metallen der Gruppen IA, IB, IIA, IIB, IIIA, IIIB, IVA, IVB, VA, VB, VIB, VIIB und VIIIB des Periodensystems, definiert gemäß Handbook of Chemistry and Physics 14th. Edition, publiziert von Chemical Rubber Publishing Co., 23 Suprior Ave. N.E., Cleveland, Ohio, enthalten, beispielsweise Halogenide, wie Chloride und Bromide, Sulfate, Phosphate, Nitrate, Borate, Alkoholate, Phenolate, Sulfonate, Oxide, Oxidhydrate, Hydroxide, Carboxilate, Chelate, Carbonate und Thio- oder Dithiocarbamate. Beispielhaft genannt seien die Kationen folgender Metalle: Lithium, Natrium, Kalium, Magnesium, Calcium, Aluminium, Gallium, Zinn, Blei, Bismut, Antimon, Kupfer, Silber, Gold, Zink, Quecksilber, Cer, Titan, Vanadium, Chrom, Molybdän, Mangan, Eisen, Cobalt und Nickel. Vorzugsweise Verwendung finden die Kationen von Lithium, Calcium, Aluminium, Zinn, Bismut, Antimon, Kupfer, Zink, Titan, Vanadium, Chrom, Molybdän, Mangan, Eisen und Cobalt. Die Katalysatoren können ohne erkennbare deutliche Nachteile auch in Form ihrer Hydrate oder Ammoniakate zum Einsatz kommen.

Als typische Katalysatoren seien beispielhaft folgende Verbindungen genannt: Lithiummethanolat, Lithiumethanolat, Lithiumpropanolat, Lithiumbutanolat, Natriummethanolat, Kalium-tert.-butanolat, Magnesiummethanolat, Calciummethanolat, Zinn-(II)-chlorid, Zinn-(IV)-chlorid, Bleiacetat, Bleiphosphat, Antimon-(III)-chlorid, Antimon-(V)-chlorid, Aluminium-isobutylat, Aluminiumtrichlorid, Bismut-(III)-chlorid, Kupfer-(II)-acetat, Kupfer-(II)-sulfat, Kupfer-(II)-nitrat, Bis-(triphenylphosphinoxido)-kupfer-(II)-chlorid, Kupfermolybdat, Silberacetat, Goldacetat, Zinkoxid, Zinkchlorid, Zinkacetat, Zinkacetonylacetat, Zinkoctoat, Zinkoxalat, Zinkhexylat, Zinkbenzoat, Zinkundecylat, Cer-(IV)-oxid, Uranylacetat, Titantetrabutanolat, Titantetrachlorid, Titantetraphenolat, Titannaphthenat, Vanadium-(III)-chlorid, Vanadiumacetonylacetat, Chrom-(III)-chlorid, Molybdän-(VI)-oxid, Molybdänacetylacetonat, Wolfram-(VI)-oxid, Mangen-(II)-acetat, Mangan-(III)-acetat, Eisen-(II)-acetat, Eisen-(III)-acetat, Eisenphosphat, Eisenoxalat, Eisen-(III)-chlorid, Eisen-(III)-bromid, Cobaltacetat, Cobaltchlorid, Cobaltsulfat, Cobaltnaphthenat, Nickelchlorid, Nickelacetat und Nickelnaphthenat sowie deren Gemische.

Es hat sich als vorteilhaft erwiesen, das entstehende Ammoniak sofort aus der Reaktionsmischung, beispielsweise durch Destillation, abzutrennen. Die hierfür verwendete Vorrichtung, beispielsweise eine Destillationskolonne, wird bei Temperaturen von 60 bis 150 °C, vorzugsweise 65 bis 120 °C betrieben, so daß eine Belegung durch Ammoniumcarbaminat, welches in minimalen Mengen aus Ammoniak und Kohlendioxid durch Zersetzung von Harnstoff gebildet wird, vermieden werden kann.

Nach beendeter Reaktion werden aus der erhaltenen Reaktionsmischung zweckmäßigerweise der Alkohol, die Dialkylcarbonate und/oder Carbamidsäurealkylester abgetrennt und zur Wiederverwendung bei nachfolgenden Ansätzen bereitgehalten; bei kontinuierlicher Fahrweise werden sie jedoch vorzugsweise direkt an den Anfang des Diurethanherstellungsprozesses zurückgeführt.

Die Abtrennung der genannten Verbindungen kann ein- oder mehrstufig durchgeführt werden. Vorzugsweise findet ein zweistufiges Verfahren Anwendung. Hierbei wird in der 1. Stufe der Alkohol bis auf einen Restalkoholgehalt von 1 bis 30 Gew.%, vorzugsweise 2 bis 15 Gew.%, bezogen auf das Gewicht der Reaktionsmischung abdestilliert und an den Prozeßbeginn zurückgeführt.

Die aufkonzentrierte Reaktionsmischung, die überwiegend aus 2-(Alkoximethyl)-pentan-1,5-diurethanen und gegebenenfalls -oligoharnstoff-polyurethanen besteht und noch den restlichen Alkohol, Dialkylcarbonat und/oder Carbamidsäurealkylester enthält, wird in der zuweiten Stufe in einer Strippkolonne mit 50 bis 5000 Liter, vorzugsweise 100 bis 1000 Liter Inertgas pro Liter aufkonzentrierter Reaktionsmischung und Stunde bei Stripptemperaturen von 50 bis 200 °C, vorzugsweise 120 bis 180 °C behandelt, um den restlichen Alkohol, die Dialkylcarbonate und/oder Carbamidsäurealkylester praktisch vollständig abzutrennen. Geeignete Inertgase hierfür sind beispielsweise Stickstoff, Kohlenmonoxid, Edelgase und Erdgase. Die abgestrippten leichtersiedenden Verbindungen werden kondensiert, gegebenenfalls zwischengelagert und zur Wiederverwendung bei weiteren Ansätzen bereitgehalten. Bei kontinuierlicher Fahrweise werden sie vorzugsweise an den Anfang des Diurethanherstellungsverfahrens zurückgeführt.

Aus der nach der Destillation oder vorzugsweise Strippung erhaltenen Reaktionsmischung, die im wesentlichen aus 2-(Alkoximethyl)-pentan-1,5-diurethanen der Formel II und gegebenenfalls 2-(Alkoximethyl)-pentan-oligoharnstoffpolyurethanen besteht, können nach bekannten Methoden, beispielsweise durch Destillation, die 2-(Alkoximethyl)-pentan-1,5-diurethane isoliert und gegebenenfalls einem zusätzlichen Reinigungsprozeß unterworfen werden.

Die im wesentlichen aus 2-(Alkoximethyl)-pentan-1,5-diurethanen und gegebenenfalls -oligoharnstoff-polyurethanen bestehende Reaktionsmischung wird jedoch vorzugsweise direkt thermisch in 2-(Alkoximethyl)-pentan-1,5-diisocyanate der Formel I und Alkohol gespalten.

Die thermische Spaltung kann in an sich bekannter Weise in der Gasphase bei Temperaturen von über 300 ° C unter vermindertem Druck, beispielsweise in Abwesenheit gelöster Katalysatoren gemäß DE-A-24 10 505 (US 3 870 739) oder in Gegenwart von Katalysatoren, beispielsweise analog den Angaben der DE-A-19 44 719 (GB 1 247 451) oder in flüssiger Phase bei Temperaturen von 175 bis 350 ° C, vorzugsweise 200 bis 280 ° C, beispielsweise in Gegenwart von Lösungsmitteln katalysatorfrei analog den Angaben der DE-A-24 21 503 (US 3 962 302) oder der DE-A-25 30 001 (US 3 919 280) oder in Gegenwart von Lösungsmitteln und Katalysatoren, beispielsweise analog den Angaben der DE-A-26 35 490 durchgeführt werden.

Die gegebenenfalls in untergeord neten Mengen 2-(Alkoximethyl)-pentan-oligoharnstoff-polyurethane enthaltende 2-(Alkoximethyl)-pentan-1,5-diurethanmischung kann in flüssiger oder in fester Form oder auch als Suspension oder Lösung in einem unter den Reaktionsbedingungen inerten Lösungsmittel in einem Verdampfer verdampft und einem nachfolgenden Spaltreaktor thermisch gespalten werden.

Nach der bevorzugten Ausführungsform des erfindungsgemäßen Verfahrens wird die Diurethanmischung lösungsmittelfrei, in Form einer auf 80 bis 180 ° C, vorzugsweise 100 bis 150 ° C erhitzten Schmelze mit einer Dosierpumpe in den Verdampfer eingebracht.

Als Verdampfer, die bei Temperaturen von 200 bis 300 ° C, vorzugsweise von 220 bis 300 ° C und insbesondere von 240 bis 280 ° C und unter einem Druck von 0,1 bis 200 mbar, vorzugsweise von 5 bis 100 mbar betrieben werden, haben sich insbesondere Dünnschichtverdampfer oder Wirbelschichtverdampfer bewährt. Es können jedoch auch beliebige andere Verdampfer verwendet werden, z.B. Schneckenverdampfer, A.P.-Reaktoren (Hersteller: Krauss-Maffei), Metallschlangen- oder Rührbett-Verdampfer.

Bei Verwendung von Dünnschichtverdampfern ist es zwar durch eine ausreichende Wärmezufuhr möglich, die gesamte zugeführte 2-(Alkoximethyl)-pentan-1,5-diurethanmischung zu verdampfen. Vorteilhafterweise wird jedoch ein Teil der zugeführten Diurethanmischung gemeinsam mit gegebenenfalls vorhandenem 2-(Alkoximethyl)-pentan-oligoharnstoff-polyurethan unverdampft als Schmelze aus dem Verdampfer ausgeschleust, da man hierdurch einen bedeutenden Reinigungseffekt an der Verdampferwand erzielt. Das Gewichtsverhältnis von verdampftem zu unverdampften 2-Alkoximethyl-pentan-1,5-diurethanen kann in breiten Grenzen, beispielsweise von 20:80 bis 90:10 variiert werden. Die aus dem Verdampfer ausgeschleuste Schmelze wird vorzugsweise direkt an den Anfang des Diurethanherstellungsprozesses, die Diurethanisierungsstufe, zurückgeführt.

Die 2-(Alkoximethyl)-pentan-1,5-diurethan-Dämpfe werden in den Spaltreaktor eingebracht und bei einer Temperatur von über 300 ° C, vorzugsweise 310 bis 480 ° C und insbesondere 350 bis 450 ° C und unter vermindertem Druck, beispielsweise von 0,1 bis 200 mbar, vorzugsweise 0,1 bis 100 mbar und insbesondere 1 bis 50 mbar, diskontinuierlich oder vorzugsweise kontinuierlich in 2-(Alkoximethyl)-pentan-1,5-diisocyanate der Formel (I) und Alkohol thermisch gespalten.

Der Spaltreaktor, der im allgemeinen säulenförmig ist, kann einen Querschnitt in beliebiger Form aufweisen. Vorzugsweise verwendet man langgestreckte, zylinderförmige Spaltreaktoren. Das Verhältnis von Innendurchmesser zu Länge des Spaltreaktors beträgt im allgemeinen 1:2 bis 1:1000, vorzugsweise 1:10 bis 1:500. Die Spaltreaktoren können senkrecht oder waagrecht ausgerichtet sein und auch Zwischenanlagen einnehmen. Vorzugsweise verwendet werden Röhrenöfen als Spaltreaktoren, bei denen der Rohrinnendurchmesser etwa 10 bis 100 mm und die Rohrlänge ungefähr 0,5 bis 5 m beträgt.

Zweckmäßigerweise führt man die Spaltung in Gegenwart von thermisch stabilen Reaktorfüllkörpern durch. Als Füllkörper geeignet sind alle temperaturbeständigen und gasdurchlässigen Materialien, wie z.B. Perlen, Wolle, Ringe und/oder Späne aus Kohle, Stahl, Messing, Kupfer, Zink, Aluminium, Titan, Chrom, Cobalt, Nickel und/oder Quartz. Einige dieser Materialien wie Stahl, Messing, Aluminium und Zink, haben sich besonders bewährt und werden daher bevorzugt verwendet, da sie zu besseren Spaltergebnissen führen. Hierbei ist noch ungeklärt, ob es sich um katalytische oder physikalische Effekte, wie beispielsweise eine bessere Wärmeübertragung, handelt ober ob eine synergistische Kombination beider Effekte vorliegt.

Aus dem Spaltreaktor führt man die in der Dampfphase befindlichen Dissoziationsprodukte, die nahezu ausschließlich aus 2-(Alkoximethyl)-pentan-1,5-diisocyanat und Alkohol bestehen, in eine mehrstufige, vorzugsweise zweistufige Dampfkondensationsvorrichtung. In der ersten Kondensationsstufe, die abh ängig vom Systemdruck von 0,1 bis 100 mbar bei Temperaturen von 60 bis 120 ° C betrieben wird, kondensieren die 2-(Alkoximethyl)-pentan-1,5-diisocyanate nahezu vollständig aus.

In der zweiten Kondensationsstufe wird im wesentlichen Alkohol kondensiert, der zur Diurethanherstellung zurückgeführt wird. Die Temperatur der zweiten Kondensationsstufe richtet sich nach dem Siedepunkt des zu kondensierenden Alkohols und dem Systemdruck und betragt beispielsweise 5 bis 30 ° C.

Die in der ersten Kondensationsstufe erhaltenen 2-(Alkoximethyl)-pentan-1,5-diisocyanate der Formel I werden üblicherweise einer Reindestillation unterworfen und besitzen danach eine Reinheit von über 99,5

Gew.%. Das hierbei anfallende Sumpfprodukt kann gegebenenfalls an den Anfang der Diurethanherstellung zurückgeführt werden.

Je nach Wahl der Kondensationstemperaturen und in Abhängigkeit vom Systemdruck, können in der ersten Kondensationsstufe Alkohol und in der zweiten Kondensationsstufe Diisocyanate in unterschiedlichen Mengen mitkondensiert werden. Nach einer bevorzugten Ausführungsform läßt man in der zweiten Kondensationsstufe mitkondensiertes Diisocyanat mit überschüssigem Alkohol zu 2-(Alkoximethyl)-pentan-1,5-diurethanen abreagieren und führt es nach dem Abtrennen von Alkohol erneut der Verdampfung und Spaltung zu. Es ist jedoch auch möglich nach einer ebenfalls bevorzugten Ausführungsform, die Diurethane mit dem Dialkylcarbonat und/oder Carbamidsäurealkylester an den Anfang des Diurethanherstellungsverfahren zurückzuführen.

Auf analoge Weise läßt man in der ersten Kondensationsstufe mitkondensierten Alkohol mit überschüssigem Diisocyanat abreagieren und führt die Reaktionsprodukte nach der destillativen Abtrennung des Diisocyanates der Verdampfung und Spaltung zu oder in der bevorzugten Ausführungsform unter Vermischung mit dem in der zweiten Kondensationsstufe erhaltenen Alkohol an den Beginn des Diurethanherstellungsverfahrens zurück.

Die neuen 2-(Alkoximethyl)-pentan-1,5-diisocyanate der Formel I sind wertvolle Ausgangsstoffe zur Herstellung von Polyurethan-, Polyharnstoff-, Polyurethan-Polyharnstoff-Kunststoffen, beispielsweise Lacken, Überzugs-, Dichtungsmassen, Klebstoffen, Elastomeren, Fasern, Bodenbelägen, Schaumstoffen u.a. nach dem Polyisocyanat-polyadditionsverfahren. Durch die Auswahl des Restes R können die physikalischen Eigenschaften wie z.B Siedepunkt, Dampfdruck, Polarität, Löslichkeit modifiziert und den örtlichen Verarbeitungsbedingungen angepaßt und gegebenenfalls die mechanischen Eigenschaften der erhaltenen Kunststoffe eingestellt, variiert und verbessert werden. Die Produkte eignen sich insbesondere zur Herstellung von lichtbeständigen Polyurethanlacken und -überzügen.

Die neuen 2-(Alkoximethyl)-pentan-1,5-diurethane der Formel II sind wertvolle End- und Zwischenprodukte.

Sie können beispielsweise als Schädlingsbekämpfungsmittel eingesetzt werden. Als Zwischenprodukte werden sie als Aufbaukomponente für Polykondensationssysteme, beispielsweise durch Reaktion mit nieder- und/oder höhermolekularen Polyhydroxylverbindungen und/oder Polyaminen zur Herstellung von Kunststoffen oder -fasern verwendet. Vorzugsweise Anwendung finden sie zur Herstellung von Diisocyanaten durch thermische Spaltung.

Die neuen 2-(Alkoximethyl)-pentan-1,5-dicarbamidsäurechloride sind insbesondere geeignet zur Herstellung von Diisocyanaten.

Die Erfindung wird durch die nachstehenden Beispiele zusätzlich erläutert.

## Beispiel 1

In einem Edelstahlautoklaven von 300 ml Inhalt wurden 12,5 g 2-(Butoximethyl)-glutarsäuredinitril, 70 ml Tetrahydrofuran und 10 g Raney-Kobalt vorgelegt und gerührt. Nach Aufpressen von 50 ml flüssigem Ammoniak wurde der Druck mit Wasserstoff auf 140 bar erhöht und der Autoklav auf 110 °C aufgeheizt. Durch Nachpressen wurde der Wasserstoffdruck auf 160 bar konstant gehalten. Nach 10 Stunden bei 110 °C wurde abgekühlt, entspannt und der Reaktionsaustrag gaschromatographisch analysiert. Die Ausbeute an 2-(Butoximethyl)-pentan-1,5-diamin betrug bei vollständigem Umsatz 85 % der Theorie. Durch fraktionierende Destillation erhielt man reines 2-(Butoximethyl)-pentan-1,5-diamin vom Siedepunkt 84 bis 86 °C/0,3 mbar.

## Beispiel 2

120 g 2-(Hexoximethyl)-glutarsäuredinitril, 900 ml Tetrahydrofuran und 70 g Raney-Kobalt wurden in einem Edelstahlautoklaven mit 200 ml flüssigem Ammoniak vereinigt, unter Rühren und unter Wasserstoff 5 Stunden auf 110 °C und 10 h auf 120 °C erhitzt. Durch Nachpressen von Wasserstoff hielt man den Druck zwischen 150 und 170 bar. Nach dem Abkühlen und Entspannen wurde der Reaktionsaustrag gaschromatographisch analysiert. Die Ausbeute an 2-(Hexoximethyl)-pentan-1,5-diamin lag bei vollständigem Umsatz bei 76 % der Theorie. Durch fraktionierende Destillation erhielt man reines 2-(Hexoximethyl)-pentan-1,5-diamin vom Siedepunkt 110 bis 115 °C/0,6 mm.

## Beispiel 3

Ein Rohrreaktor wurde mit 178 ml eines Eisenkatalysators, der durch Reduktion von Eisenoxiden mit

Wasserstoff bei einer Temperatur von etwa 500 °C erhalten wurde (z.B. nach DE-A 24 29 293), gefüllt. In Rieselfahrweise wurden dann stündlich 10 g 2-(Butoximethyl)-glutarsäuredinitril, 90 g Tetrahydrofuran, 6,5 g flüssiges Ammoniak, 10 l rohes Hydriergemisch (Kreislauf-Fahrweise) sowie Wasserstoff zugeführt. Hydriert wurde bei einer Temperatur von 160 °C und einem Druck von 100 bar. Nach dem Verdampfen des Ammoniaks wurde das rohe Hydrierprodukt gaschromatographisch analysiert: Die Ausbeute an 2-(Butoximethyl)-pentan-1,5-diamin, bezogen auf umgesetztes Edukt, betrug 74 % der Theorie.

Beispiel 4

91 g 2-(2-Methoxiethoximethyl)-glutarsäuredinitril, 800 ml Tetrahydrofuran, 90 g Raney-Kobalt und 200 ml flüssiges Ammoniak wurden in einem Edelstahlautoklaven unter Rühren und unter Wasserstoff 5 h auf 110 °C und 10 h auf 120 °C erhitzt. Durch Nachpressen von Wasserstoff wurde der Druck auf 150 bar konstant gehalten. Nach dem Abkühlen und Entspannen wurde der Reaktionsaustrag gaschromatographisch analysiert. Die Ausbeute an 2-(2-Methoxiethoximethyl)-pentan-1,5-diamin betrug bei vollständigem Umsatz 68 % der Theorie. Durch fraktionierende Destillation erhielt man reines 2-(2-Methoxiethoximethyl)-pentan-1,5-diamin vom Siedepunkt 105 bis 107 °C/0,3 mbar.

Beispiel 5

In einem 1-l-Rührautoklaven mit aufgesetzter Druckkolonne und Druckregelventil wurden 188 g 2-(Butoximethyl)-pentan-1,5-diamin, 126 g Harnstoff und 370 g Butanol 4 Stunden bei 230 °C und 9 bar unter Abnahme von Ammoniak am Rückfluß gekocht. Man erhielt 609 g einer klaren Flüssigkeit, deren Analyse mittels Gelpermeationschromatographie eine Umwandlung in 2-(Butoximethyl)-1,5-bis-(butoxicarbonylamino)-pentan von 95 % anzeigte. Nach Abdestillieren von überschüssigem Butanol und geringen Mengen Carbamidsäurebutylester verblieben 374 g einer zähigen Flüssigkeit, die ohne weitere Reinigung in die thermische Spaltung eingesetzt wurde. Durch Säulenchromatographie an Kieselgel erhielt man reines 2-(Butoximethyl)-1,5-bis-(butoxi-carbonylamino)-pentan als farbloses, zähes Öl.

| C,H,N-Analyse: | | | |
|---|---|---|---|
| | C | H | N |
| berechnet | 61,82 % | 10,38 % | 7,21 % |
| gefunden | 62,04 % | 10,19 % | 7,34 % |

Beispiel 6

Eine Spaltapparatur, bestehend aus einem Dünnschichtverdampfer, einem Spaltreaktor (zylindrisches Rohr aus V2A-Stahl von ca. 1 l Leervolumen bestückt mit verzinkten Blechfüllkörpern) und einer zweistufigen Dampfkondensationsvorrichtung, wurde auf 5 mbar evakuiert. Innerhalb von 2 Stunden wurden 350 g des nach Beispiel 5 erhaltenen 2-(Butoximethyl)-1,5-bis(butoxicarbonylamino)-pentans in den auf 260 °C erhitzten Dünnschichtverdampfer eingebracht, wovon 315 g verdampften und 35 g abliefen. Die Diurethandämpfe gelangten in den Spaltreaktor, dessen durchschnittliche Temperatur 400 °C betrug. Die austretenden Spaltgase wurden in einer nachgeschalteten zweistufigen Kondensationsvorrichtung bei 65 bis 18 °C fraktionierend kondensiert. Im ersten Kondensator fielen 217 g rohes Diisocyanat an, das durch Vakuumdestillation (Übergangstemperatur 130 bis 132 °C/0,3 mbar) gereinigt wurde. Man erhielt 125,3 g (63 % Ausbeute) 2-(Butoximethyl)-pentan-1,5-diisocyanat mit einer Reinheit von 98 %.

Beispiel 7

In einem 1-l-Rührautoklaven mit aufgesetzter Druckkolonne und Druckregelventil wurden 216 g 2-(Hexoximethyl)-pentan-1,5-diamin, 126 g Harnstoff und 444 g Butanol 5 Stunden bei 230 °C und 10 bar unter Abnahme von Ammoniak am Rückfluß gekocht. Man erhielt 702 g einer klaren Flüssigkeit, deren Analyse mittels Gelpermeationschromatographie eine Umwandlung in 2-(Hexoximethyl)-1,5-bis-(butoxicarbonylamino)-pentan von 97 % anzeigte. Nach Abdestillieren von überschüssigem Butanol und geringen Mengen Carbamidsäurebutylester verblieben 398 g einer zähen Flüssigkeit, die ohne weitere Reinigung in die thermische Spaltung eingesetzt wurde. Durch Säulenchromatographie an Kieselgel erhielt

man reines 2-(Hexoximethyl)-1,5-bis(butoxicarbonylamino)-pentan als farbloses, zähes Öl.

| C,H,N-Analyse: | | | |
|---|---|---|---|
| | C | H | N |
| berechnet<br>gefunden | 63,43 %<br>63,48 % | 10,65 %<br>10,79 % | 6,72 %<br>6,60 % |

Beispiel 8

Die Spaltung wurde in der in Beispiel 6 beschriebenen Apparatur durchgeführt. 550 g des nach Beispiel 7 erhaltenen 2-(Hexoximethyl)-1,5-bis(butoxicarbonylamino)-pentans wurden innerhalb von 3 Stunden in den auf 280 °C beheizten und auf 8 bis 9 mbar evakuierten Dünnschichtverdampfer eingebracht, wobei 509 g verdampften und 41 g abliefen. Die Temperatur im Spaltreaktor betrug durchschnittlich 400 °C. Im bei 85 °C betriebenen Kondensator wurden 343 g rohes Isocyanat gesammelt. Dieses wurde durch Destillation über einen Dünnschichtverdampfer (Öltemperatur 160 °C/0,2 mbar) vorgereinigt und nochmals unter vermindertem Druck bei 123 bis 125 °C/0,2 mbar destilliert. Man erhielt 207 g (65 % Ausbeute) 2-(Hexoximethyl)-pentan-1,5-diisocyanat mit einer Reinheit von 99 %.

Beispiel 9

In einem 1-l-Rührautoklaven mit aufgesetzter Druckkolonne und Druckregelventil wurden 190 g 2-(2-Methoxiethoximethyl)-pentan-1,5-diamin, 126 g Harnstoff und 444 g Butanol 6 Stunden bei 230 °C und 10 bar unter Abnahme von Ammoniak am Rückfluß gekocht. Man erhielt 685 g einer klaren, gelblichen Flüssigkeit, deren Analyse mittels Gelpermeationschromatographie eine Umwandlung in 2-(2-Methoxiethoximethyl)-1,5-bis(butoxicarbonylamino)-pentan von 96 % anzeigte. Nach Abdestillieren von überschüssigem Butanol und geringen Mengen Carbamidsäurebutylester verblieben 380 g einer zähen Flüssigkeit, die ohne weitere Reinigung in die thermische Spaltung eingesetzt wurde.

Durch Säulenchromatographie am Kieselgel erhielt man reines 2-(2-Methoxiethoximethyl)-1,5-bis-(butoxicarbonylamino)-pentan als farbloses, zähes Öl.

| C,H,N-Analyse: | | | |
|---|---|---|---|
| | C | H | N |
| berechnet<br>gefunden | 60,93 %<br>60,66 % | 10,23 %<br>10,29 % | 7,48 %<br>7,38 % |

Beispiel 10

Zu einer auf 0 °C abgekühlten Mischung aus 200 g o-Dichlorbenzol und 60 g Phosgen tropfte man unter kräftigem Rühren und Eiskühlung 18,8 g 2-(Butoximethyl)-pentan-1,5-diamin. Nach beendeter Zugabe wurde die entstandene Suspension auf 130 °C erwärmt und bei dieser Temperatur 2,5 Stunden Phosgen durch das Reaktionsgemisch geleitet. Nach dem Abkühlen wurde das überschüssige Phosgen mit einem kräftigen Stickstoffstrom ausgetrieben, das o-Dichlorbenzol unter vermindertem Druck bei 10 mbar abdestilliert und der Rückstand bei 125 bis 128 °C bei 0,2 mbar destilliert. Man erhielt 18,7 g (78 % Ausbeute) 2-Butoximethyl-pentan-1,5-diisocyanat.

**Patentansprüche**

**1.** 2-(Alkoximethyl)-pentan-1,5-diisocyanate der Formel (I)

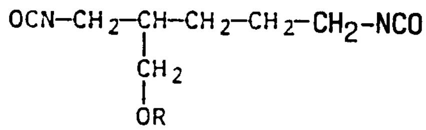

(I),

in der R bedeutet:
einen geradkettigen oder verzweigten $C_1$-$C_{20}$-Alkylrest,
einen geradkettigen oder verzweigten $C_2$-$C_{20}$-Alkenylrest,
einen geradkettigen oder verzweigten $C_3$-$C_{20}$-Oxaalkylrest,
einen $C_5$-$C_{12}$-Cycloalkylrest oder
einen $C_7$-$C_{20}$-Aralkylrest.

**2.** 2-(Alkoximethyl)-pentan-1,5-diurethane der Formel (II)

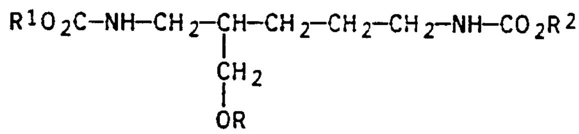

(II),

in der

| | |
|---|---|
| R | ein geradkettiger oder verzweigter $C_1$-$C_{20}$-Alkylrest,<br>ein geradkettiger oder verzweigter $C_2$-$C_{20}$-Alkenylrest,<br>ein geradkettiger oder verzweigter $C_3$-$C_{20}$-Oxaalkylrest,<br>ein $C_5$-$C_{12}$-Cycloalkylrest oder<br>ein $C_7$-$C_{20}$-Aralkylrest ist<br>und |
| $R^1$ und $R^2$ | gleich oder verschieden sind und einen geradkettigen oder verzweigten $C_1$-$C_{20}$-Alkylrest oder einen $C_3$-$C_{15}$-Cycloalkylrest bedeuten. |

**3.** 2-(Alkoximethyl)-pentan-1,5-dicarbamidsäurechloride der Formel (III)

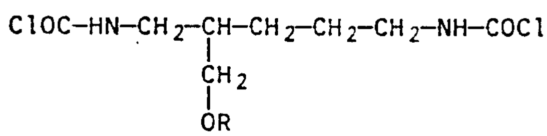

(III),

in der R bedeutet:
einen geradkettigen oder verzweigten $C_1$-$C_{20}$-Alkylrest,
einen geradkettigen oder verzweigten $C_2$-$C_{20}$-Alkenylrest,
einen geradkettigen oder verzweigten $C_3$-$C_{20}$-Oxaalkylrest,
einen $C_5$-$C_{12}$-Cycloalkylrest oder
einen $C_7$-$C_{20}$-Aralkylrest.

**4.** Verfahren zur Herstellung von 2-(Alkoximethyl)-pentan-1,5-diisocyanaten der Formel (I)

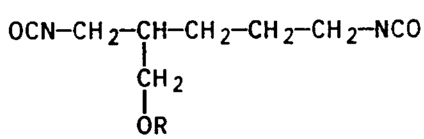

(I),

in der R bedeutet:
einen geradkettigen oder verzweigten $C_1$-$C_{20}$-Alkylrest,
einen geradkettigen oder verzweigten $C_2$-$C_{20}$-Alkenylrest,
einen geradkettigen oder verzweigten $C_3$-$C_{20}$-Oxaalkylrest,

einen $C_5$-$C_{12}$-Cycloalkylrest oder
einen $C_7$-$C_{20}$-Aralkylrest,

dadurch gekennzeichnet, daß man 2-(Alkoximethyl)-pentan-1,5-diurethane der Formel (II)

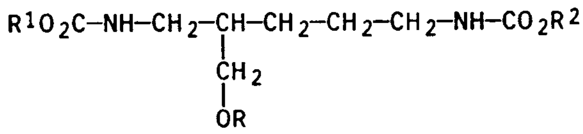
(II),

in der R die obengenannte Bedeutung aufweist und $R^1$ und $R^2$ gleich oder verschieden sind und einen geradkettigen oder verzweigten $C_1$-$C_{20}$-Alkylrest oder eine $C_3$-$C_{15}$-Cycloalkylrest bedeuten, in Gegenwart oder Abwesenheit von Katalysatoren

a) in der Gasphase bei Temperaturen von über 300 °C unter vermindertem Druck oder
b) in flüssiger Phase bei Temperaturen von 175 bis 350 °C thermisch spaltet.

**5.** Verfahren zur Herstellung von 2-(Alkoximethyl)-pentan-1,5-diisocyanaten der Formel (I)

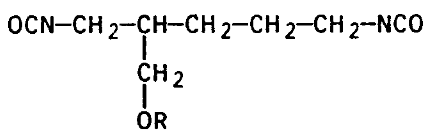
(I),

in der R bedeutet:
einen geradkettigen oder verzweigten $C_1$-$C_{20}$-Alkylrest,
einen geradkettigen oder verzweigten $C_2$-$C_{20}$-Alkenylrest,
einen geradkettigen oder verzweigten $C_3$-$C_{20}$-Oxaalkylrest,
einen $C_5$-$C_{12}$-Cycloalkylrest oder
einen $C_7$-$C_{20}$-Aralkylrest,
dadurch gekennzeichnet, daß man 2-(Alkoximethyl)-pentan-1,5-dicarbamidsäurechloride der Formel (III)

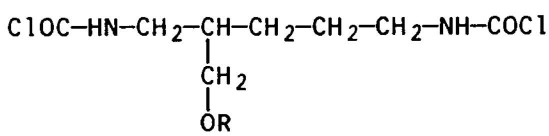
(III),

in der R die obengenannte Bedeutung besitzt, in Gegenwart von unter den Reaktionsbedingungen gegenüber NCO-Gruppen inerten organischen Lösungsmitteln bei Temperaturen von 80 bis 200 °C thermisch spaltet.

**6.** Verfahren zur Herstellung von 2-(Alkoximethyl)-pentan-1,5-diurethanen der Formel (II)

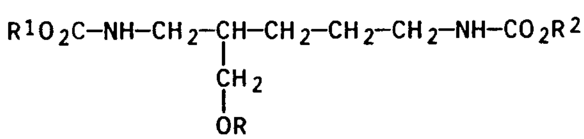
(II),

in der
R ein geradkettiger oder verzweigter $C_1$-$C_{20}$-Alkylrest,
ein geradkettiger oder verzweigter $C_2$-$C_{20}$-Alkenylrest,
ein geradkettiger oder verzweigter $C_3$-$C_{20}$-Oxaalkylrest,
ein $C_5$-$C_{12}$-Cycloalkylrest oder
ein $C_7$-$C_{20}$-Aralkylrest ist

und

$R^1$ und $R^2$ gleich oder verschieden sind und einen geradkettigen oder verzweigten $C_1$-$C_{20}$-Alkylrest oder einen $C_3$-$C_{15}$-Cycloalkylrest bedeuten,

dadurch gekennzeichnet, daß man 2-(Alkoximethyl)-pentan-1,5-diamine der Formel (IV)

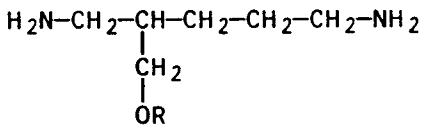

(IV),

in der R die obengenannte Bedeutung besitzt in Gegenwart oder Abwesenheit von Katalysatoren mit

a) Harnstoff und einem primären und/oder sekundären Alkohol $R^1OH$ bzw. $R^2OH$ oder

b) Harnstoff und einem primären und/oder sekundären Alkohol $R^1OH$ bzw. $R^2OH$, wobei die Reste $R^1$ und $R^2$ gleich oder verschieden sind und einen geradkettigen oder verzweigten $C_1$-$C_{20}$-Alkylrest oder einen $C_3$-$C_{15}$-Cycloalkylrest bedeuten, in Gegenwart von Carbamidsäurealkylestern und/oder Dialkylcarbonaten umsetzt und den entstehenden Ammoniak gegebenenfalls abtrennt.

**7.** Verfahren zur Herstellung von 2-(Alkoximethyl)-pentan-1,5-diurethanen der Formel (II) nach Anspruch 6, dadurch gekennzeichnet, daß man die 2-(Alkoximethyl)-pentan-1,5-diamine mit Harnstoff und Alkohol im Molverhältnis 1:1,5 bis 10:2 bis 50 umsetzt.

**8.** Verfahren zur Herstellung von 2-(Alkoximethyl)-pentan-1,5-diurethanen der Formel (II) nach Anspruch 6, dadurch gekennzeichnet, daß man den dem Alkohol entsprechenden Carbamidsäurealkylester in einer Menge von 1 bis 20 Mol.%, bezogen auf 2-(Alkoximethyl)-pentan-1,5-diamin, verwendet.

**9.** Verwendung von 2-(Alkoximethyl)-pentan-1,5-diurethanen der Formel (II) nach Anspruch 2 zur Herstellung von 2-(Alkoximethyl)-pentan-1,5-diisocyanaten.

**10.** Verwendung von 2-(Alkoximethyl)-pentan-1,5-diisocyanaten der Formel (I) nach Anspruch 1 zur Herstellung von Kunststoffen nach dem Polyisocyanat-polyadditionsverfahren.

## Claims

**1.** A 2-(alkoxymethyl)pentane 1,5-diisocyanate of the formula (I)

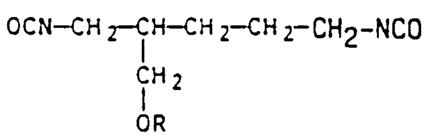

(I)

where R is
straight-chain or branched $C_1$-$C_{20}$-alkyl,
straight-chain or branched $C_2$-$C_{20}$-alkenyl,
straight-chain or branched $C_3$-$C_{20}$-oxaalkyl,
$C_5$-$C_{12}$-cycloalkyl or
$C_7$-$C_{20}$-aralkyl.

**2.** A 2-(alkoxymethyl)pentane-1,5-diurethane of the formula (II)

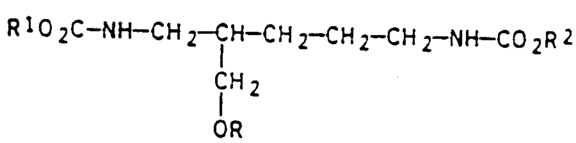

(II)

where R is

straight-chain or branched $C_1$-$C_{20}$-alkyl,
straight-chain or branched $C_2$-$C_{20}$-alkenyl,
straight-chain or branched $C_3$-$C_{20}$-oxaalkyl,
$C_5$-$C_{12}$-cycloalkyl or
$C_7$-$C_{20}$-aralkyl,
and
$R^1$ and $R^2$ are identical or different and are straight-chain or branched $C_1$-$C_{20}$-alkyl or $C_3$-$C_{15}$-cycloalkyl.

**3.** A 2-(alkoxymethyl)pentane-1,5-dicarbamoyl chloride of the formula (III)

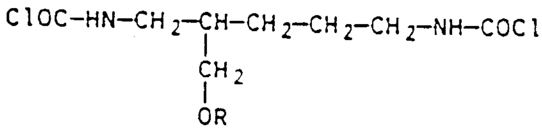

(III)

where R is
straight-chain or branched $C_1$-$C_{20}$-alkyl,
straight-chain or branched $C_2$-$C_{20}$-alkenyl,
straight-chain or branched $C_3$-$C_{20}$-oxaalkyl,
$C_5$-$C_{12}$-cycloalkyl or
$C_7$-$C_{20}$-aralkyl.

**4.** A process for the preparation of a 2-(alkoxymethyl)pentane 1,5-diisocyanate of the formula (I)

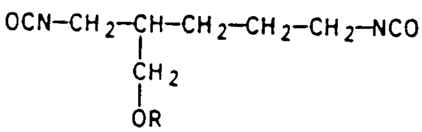

(I)

where R is
straight-chain or branched $C_1$-$C_{20}$-alkyl,
straight-chain or branched $C_2$-$C_{20}$-alkenyl,
straight-chain or branched $C_3$-$C_{20}$-oxaalkyl,
$C_5$-$C_{12}$-cycloalkyl or
$C_7$-$C_{20}$-aralkyl,
which comprises pyrolyzing a 2-(alkoxymethyl)pentane-1,5-diurethane of the formula (II)

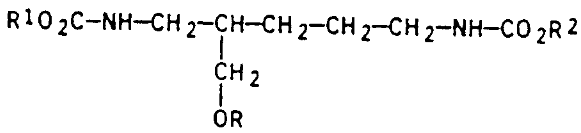

(II)

where R is as defined above, and $R^1$ and $R^2$ are identical or different and are straight-chain or branched $C_1$-$C_{20}$-alkyl or $C_3$-$C_{15}$-cycloalkyl,
in the presence or absence of a catalyst,
a) in the gas phase at above 300 °C under reduced pressure, or
b) in the liquid phase at from 175 to 350 °C.

**5.** A process for the preparation of a 2-(alkoxymethyl)pentane 1,5-diisocyanate of the formula (I)

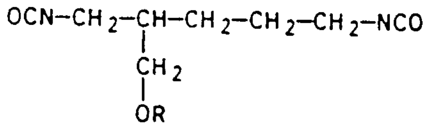

(I)

where R is
straight-chain or branched $C_1$-$C_{20}$-alkyl,
straight-chain or branched $C_2$-$C_{20}$-alkenyl,
straight-chain or branched $C_3$-$C_{20}$-oxaalkyl,
$C_5$-$C_{12}$-cycloalkyl or
$C_7$-$C_{20}$-aralkyl,
which comprises pyrolyzing a 2-(alkomethyl)pentane-1,5-dicarbamoyl chloride of the formula (III)

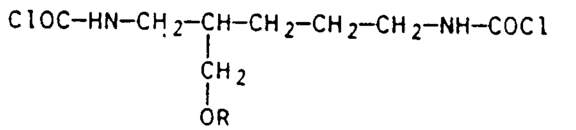

(III)

where R is as defined above, at from 80 to 200 °C in the presence of an organic solvent which is inert toward NCO groups under the reaction conditions.

6. A process for the preparation of a 2-(alkoxymethyl)pentane-1,5-diurethane of the formula (II)

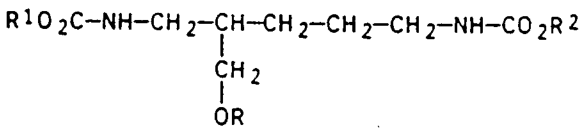

(II)

where R is
straight-chain or branched $C_1$-$C_{20}$-alkyl,
straight-chain or branched $C_2$-$C_{20}$-alkenyl,
straight-chain or branched $C_3$-$C_{20}$-oxaalkyl,
$C_5$-$C_{12}$-cycloalkyl or
$C_7$-$C_{20}$-aralkyl,
and
$R^1$ and $R^2$ are identical or different and are straight-chain or branched $C_1$-$C_{20}$-alkyl or $C_3$-$C_{15}$-cycloalkyl,
which comprises reacting a 2-(alkoxymethyl)pentane-1,5-diamine of the formula (IV)

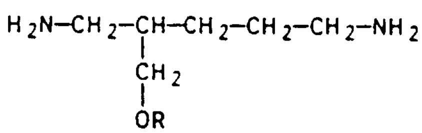

(IV)

where R is as defined above, in the presence or absence of a catalyst, with
a) urea and a primary and/or secondary alcohol $R^1OH$ or $R^2OH$, or
b) urea and a primary and/or secondary alcohol $R^1OH$ or $R^2OH$ where $R^1$ and $R^2$ are identical or different and are straight-chain or branched $C_1$-$C_{20}$-alkyl or $C_3$-$C_{15}$-cycloalkyl, in the presence of an alkyl carbamate and/or dialkyl carbonate, and, if desired, removing the ammonia formed.

7. A process for the preparation of a 2-(alkoxymethyl)pentane-1,5-diurethane of the formula (II) as claimed in claim 6, wherein the 2-(alkoxymethyl)pentane-1,5-diamine is reacted with urea and alcohol in a molar ratio of from 1:1.5 to 10:2 to 50.

**8.** A process for the preparation of a 2-(alkoxymethyl)pentane-1,5-diurethane of the formula (II) as claimed in claim 6, wherein the alkyl carbamate corresponding to the alcohol is used in an amount of from 1 to 20 mol.%, based on 2-(alkoxymethyl)pentane-1,5-diamine.

**9.** The use of a 2-(alkoxymethyl)pentane-1,5-diurethane of the formula (II) as claimed in claim 2 for the preparation of a 2-(alkoxymethyl)pentane 1,5-diisocyanate.

**10.** The use of a 2-(alkoxymethyl)pentane 1,5-diisocyanate of the formula (I) as claimed in claim 1 for the preparation of plastics by the polyisocyanate polyaddition process.

## Revendications

**1.** 2-(alcoxyméthyl)-pentane-1,5-diisocyanates de formule (I)

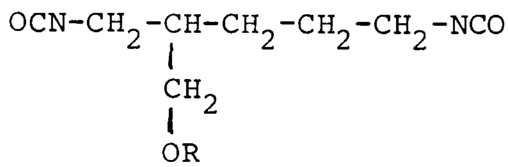

(I)

dans laquelle R représente :
un reste alkyle en $C_1$-$C_{20}$ à chaîne droite ou ramifiée,
un reste alcényle en $C_2$-$C_{20}$ à chaîne droite ou ramifiée,
un reste oxaalkyle en $C_3$-$C_{20}$ à chaîne droite ou ramifiée,
un reste cycloalkyle en $C_5$-$C_{12}$ ou
un reste aralkyle en $C_7$-$C_{20}$.

**2.** 2-(alcoxyméthyl)-pentane-1,5-diuréthannes de formule (II)

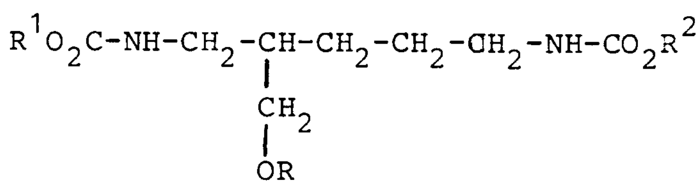

(II)

dans laquelle

R est un reste alkyle en $C_1$-$C_{20}$ à chaîne droite ou ramifiée,
un reste alcényle en $C_2$-$C_{20}$ à chaîne droite ou ramifiée,
un reste oxaalkyle en $C_3$-$C_{20}$ à chaîne droite ou ramifiée,
un reste cycloalkyle en $C_5$-$C_{12}$ ou
un reste aralkyle en $C_7$-$C_{20}$

et

$R^1$ et $R^2$ sont identiques ou différents et représentent un reste alkyle en $C_1$-$C_{20}$ à chaîne droite ou ramifiée ou un reste cycloalkyle en $C_3$-$C_{15}$.

**3.** Chlorures d'acides 2-(alcoxyméthyl)-pentane-1,5-dicarbamiques de formule (III)

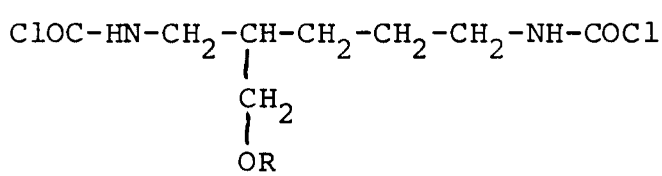

(III)

dans laquelle R représente :
un reste alkyle en $C_1$-$C_{20}$ à chaîne droite ou ramifiée,
un reste alcényle en $C_2$-$C_{20}$ à chaîne droite ou ramifiée,

un reste oxaalkyle $C_3$-$C_{20}$ à chaîne droite ou ramifiée,
un reste cycloalkyle en $C_5$-$C_{12}$ ou
un reste aralkyle en $C_7$-$C_{20}$.

**4.** Procédé de préparation de 2-(alcoxyméthyl)-pentane-1,5-diisocyanates de formule (I)

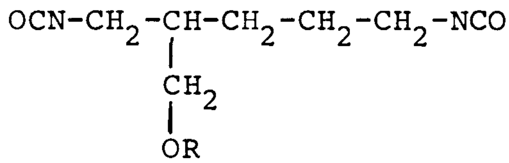

(I)

dans laquelle R représente :
un reste alkyle en $C_1$-$C_{20}$ à chaîne droite ou ramifiée,
un reste alcényle en $C_2$-$C_{20}$ à chaîne droite ou ramifiée,
un reste oxaalkyle en $C_3$-$C_{20}$ à chaîne droite ou ramifiée,
un reste cycloalkyle en $C_5$-$C_{12}$ ou
un reste aralkyle en $C_7$-$C_{20}$,
caractérisé en ce que l'on dissocie par voie thermique
a) en phase gazeuse à des températures supérieures à 300 °C et sous pression réduite ou
b) en phase liquide à des températures de 175 à 350 °C,
des 2-(alcoxyméthyl)-pentane-1,5-diuréthannes de formule (II)

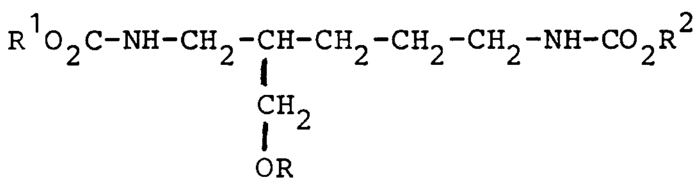

(II)

dans laquelle R a les significations sus-indiquées et $R^1$ et $R^2$ sont identiques ou différents et représentent un reste alkyle en $C_1$-$C_{20}$ à chaîne droite ou ramifiée ou un reste cycloalkyle en $C_3$-$C_{15}$, en présence ou en l'absence de catalyseurs.

**5.** Procédé de préparation de 2-(alcoxyméthyl)-pentane-1,5-diisocyana

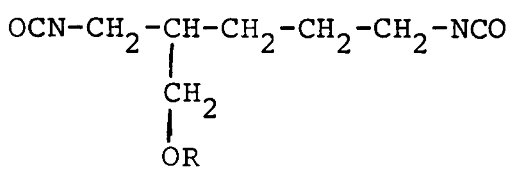

(I)

tes de formule (I) dans laquelle R représente :
un reste alkyle en $C_1$-$C_{20}$ à chaîne droite ou ramifiée,
un reste alcényle en $C_2$-$C_{20}$ à chaîne droite ou ramifiée,
un reste oxaalkyle en $C_3$-$C_{20}$ à chaîne droite ou ramifiée,
un reste cycloalkyle en $C_5$-$C_{12}$ ou
un reste aralkyle en $C_7$-$C_{20}$,
caractérisé en ce que l'on dissocie par voie thermique à des températures de 80 à 200 °C des chlorures d'acides 2-(alcoxyméthyl)-pentane-1,5-dicarbamiques de formule (III)

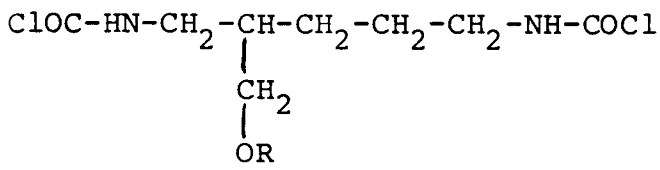

(III)

dans laquelle R a les significations sus-indiquées, en présence de solvants organiques inertes vis à vis des groupes NCO dans les conditions de la réaction.

**6.** Procédé de préparation de 2-(alcoxyméthyl)-pentane-1,5-diuréthannes de formule (II)

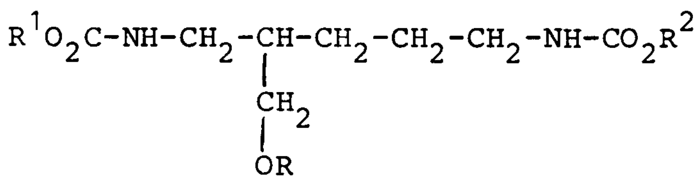

(II)

dans laquelle R est
un reste alkyle en $C_1$-$C_{20}$ à chaîne droite ou ramifiée,
un reste alcényle en $C_2$-$C_{20}$ à chaîne droite ou ramifiée,
un reste oxaalkyle en $C_3$-$C_{20}$ à chaîne droite ou ramifiée,
un reste cycloalkyle en $C_5$-$C_{12}$ ou
un reste aralkyle en $C_7$-$C_{20}$,
et

$R^1$ et $R^2$ sont identiques ou différents et représentent un reste alkyle en $C_1$-$C_{20}$ à chaîne droite ou ramifiée, ou un reste cycloalkyle en $C_3$-$C_{15}$,

caractérisé en ce que l'on fait réagir des 2-(alcoxyméthyl)pentane-1,5-diamines de formule(IV)

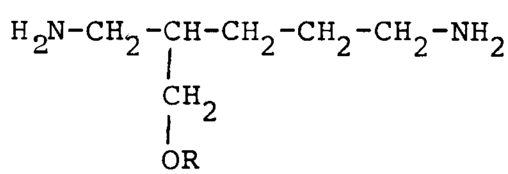

(IV)

dans laquelle R a les significations sus-indiquées en présence ou en l'absence de catalyseurs avec

a) de l'urée et un alcool primaire et/ou secondaire $R^1OH$ ou $R^2OH$ ou

b) de l'urée et un alcool primaire et/ou secondaire $R^1OH$ ou $R^2OH$, les restes $R^1$ et $R^2$ étant identiques ou différents et représentant un reste alkyle en $C_1$-$C_{20}$ à chaîne droite ou ramifiée ou un reste cycloalkyle en $C_3$-$C_{15}$, en présence d'esters alkyliques de l'acide carbamique et/ou de carbonates dialkyliques

et en ce que l'on sépare éventuellement l'ammoniac qui se dégage.

**7.** Procédé de préparation de 2-(alcoxyméthyl)-pentane-1,5-diuréthannes de formule (II) suivant la revendication 6, caractérisé en ce que l'on fait réagir les 2-(alkyloxyméthyl)-pentane-1,5-diamines avec l'urée et l'alcool dans un rapport molaire de 1:1,5 à 10:2 à 50.

**8.** Procédé de préparation de 2-(alcoxyméthyl)-pentane-1,5-diuréthannes de formule (II) suivant la revendication 6, caractérisé en ce que l'on utilise l'ester alkylique d'acide carbamique correspondant à l'alcool en une quantité de 1 à 20 moles pour cent, par rapport à la 2-(alcoxyméthyl )-pentane-1,5-diamine.

**9.** Utilisation des 2-(alcoxyméthyl )-pentane-1,5-diuréthannes de la formule (II) suivant la revendication 2 pour la préparation de 2-(alcoxyméthyl)-pentane-1,5-diisocyanates.

**10.** Utilisation des 2-(alcoxyméthyl )-pentane-1,5-diisocyanates de la formule (I) suivant la revendication 1 pour la fabrication de matières plastiques suivant le procédé d'obtention de polyisocyanates par

polyaddition.